# EUROPÄISCHE PATENTANMELDUNG

(11) **EP 0 534 345 A2**
(43) Veröffentlichungstag der Anmeldung: **31.03.1993**
(21) Anmeldenummer: 92116113.9
(22) Anmeldetag: 21.09.1992
(51) Int. Cl.: C12Q 1/68, C12N 15/10, C07H 21/00

(54) **Verfahren zur spezifischen Herstellung von Ribonukleinsäuren**

(30) Priorität: 26.09.1991 DE 4132133
(71) Anmelder: BOEHRINGER MANNHEIM GMBH, D-68298 Mannheim (DE)
(72) Erfinder: Reischl, Udo, W-8352 Grafenau (DE); Rüger, Rüdiger, Dr. rer. nat., W-8124 Seeshaupt (DE); Kessler, Christoph, Dr. rer. nat., W-8021 Dorfen (DE); Seibl, Rudolf, Dr. rer. nat., W-8122 Penzberg (DE)

(57) **Zusammenfassung**

Verfahren zur Herstellung von Ribonukleinsäuren oder zum Nachweis von Templatnukleinsäuren nach dem Transkriptionsprinzip unter Verwendung zweier mit benachbarten Bereichen der Templatnukleinsäure hybridisierbarer Oligonukleotide ohne Ligation dieser Oligonukleotide.

## Beschreibung

Gegenstand der Erfindung sind ein verfahren zur spezifischen Herstellung von Ribonukleinsäuren und ein Verfahren zum spezifischen Nachweis von Nukleinsäuren sowie Reagenzien zur Durchführung dieser beiden Verfahren.

Nukleinsäuren sind für alle bisher bekannten Organsimen als Informationsträger die Grundlage spezifischer Lebensformen. Sie codieren für Proteine, manche Nukleinsäuren haben jedoch wahrscheinlich auch katalytische oder strukturelle Wirksamkeiten. Nukleinsäuren können wegen ihrer Spezifität auch zur Unterscheidung und zum Nachweis von Organismen herangezogen werden. Die einzelnen Nukleinsäuren sind in Organismen jedoch in nur sehr begrenzter Menge vorhanden. Zur praktischen Handhabbarkeit von Nukleinsäuren hat es sich daher als günstig erwiesen, diese Nukleinsäuren in vivo (Klonierung) oder in vitro (Amplifikation) zu vermehren. Während der erste Weg zeitraubend und umständlich ist, wurde die in-vitro-Amplifikation in den letzten Jahren zu einer praktikablen Alternative entwickelt.

In der EP-A-200 362 ist ein Verfahren beschrieben, welches eine in Zyklen verlaufende Amplifikation eines Teils einer Startnukleinsäure zum Gegenstand hat. In jedem Zyklus entsteht zu den vorhandenen Nukleinsäuren jeweils ein Gegenstrang. Die Reaktionsführung hat jedoch eine relativ hohe Zyklenzahl zur Folge.

In der EP-A-0 320 308 wird eine sogenannte Ligase Chain Reaction beschrieben. Darin werden Oligonukleotide, die im hybridisierten Zustand nur durch einen sogenannten Nick getrennt sind, durch eine Ligasereaktion verknüpft. Die so hergestellte Nukleinsäure dient wiederum als Templat zur Ligierung der Gegenstrangoligonukleotide usw. . Diese Reaktion hat den gleichen Nachteil wie die PCR, nämlich daß in jedem Zyklus erneut die Doppelstränge getrennt werden müssen.

Diesen Nachteil versucht man in Verfahren, die auf Transkriptionsschritten beruhen, welche in isothermen Zyklen zu einer Vielzahl von Kopien führen, zu umgehen. Ein solches Verfahren ist beispielsweise in der EP-A-O 310 229 beschrieben. Darin wird ein Oligonukleotid (Promotorprimer), welches sowohl einen Templatespezifischen Bereich als auch eine T7-Promotorsequenz enthält, an der Templatnukleinsäure mit Mononukleotiden verlängert. Mittels eines zweiten Primers wird dann ein Gegenstrang gebildet. Dabei wird auch zu dem bislang einzelsträngigen Promotorbereich ein Gegenstrang gebildet und damit die Funktionalität des Promotors hergestellt. Anschließend findet eine Promotor-kontrollierte Transkription des gebildeten Hybrids statt. Zu den gebildeten Transkript-RNAs wird mittels eines Gegenstrangprimers cDNA hergestellt. Das Hybrid wird denaturiert und die cDNA erneut mit Promotorprimer umgesetzt. Verlängerung dieses Primers an der cDNA führt wiederum zu einem Hybrid, welches einen funktionellen Promotor enthält. Dieses Molekül kann ebenfalls in den Transkriptionszyklus eingeführt werden. Nachteilig an dieser Reaktionsführung ist die Tatsache, daß zwei Verlängerungsreaktionen zur Herstellung eines transkribierbaren Moleküls erforderlich sind. Die selben Probleme treten auch bei dem Verfahren der WO 88/10315 und der EP-A-0 329 822 sowie der EP-A-0 373 960 auf.

In der EP-A-0 427 074 wird ein Verfahren vorgeschlagen, bei dem die Templatnukleinsäure mit einem templatspezifischen promotorhaltigen Primer direkt zu einem transkribierbaren Molekül umgesetzt wird. Die anschließende Transkription ergibt RNA, welche zum einen Teil einem Teil der Templatnukleinsäure entspricht und zu einem anderen Teil komplementär einer auf dem Primer befindlichen weiteren Sequenz ist. Ein Nachteil dieses Verfahrens ist, daß die Reaktion als Nebenprodukt ohne Anwesenheit von Templatnukleinsäure dasselbe Molekül liefert wie als Hauptprodukt bei Anwesenheit der Templatnukleinsäure. Es handelt sich also um ein relativ unspezifisches Verfahren. Ebenfalls darin beschreiben ist ein Verfahren, bei dem zwei verschiedene Primer benutzt werden, welche im an die Templatnukleinsäure hybridisierten Zustand ligiert werden, wobei sich ein verlängertes transkriptierbares Molekül bildet. Ein Nachteil ist hier die Verwendung der Ligase, da sie ein zusätzliches Enzym ist, welches meist andere Reaktionsbedingungen benötigt als die RNA-Polymerase, mit der die anschließende Transkription stattfindet.

In der EP-A-0 427 073 ist eine weitere Reaktion zur Synthese einer transkribierbaren Nukleinsäure beschrieben. Dabei wird die Templatnukleinsäure an ihrem 3'-Ende mit dem 5'-Ende eines Promotorprimers ligiert. Durch Hybridisierung mit der templatspezifischen Sequenz des überstehenden 3'-Endes wird das Templat an den Promotorprimer gebunden. Diese Reaktion benötigt daher ebenfalls eine enzymatische Reaktion (Ligase) zur Herstellung des transkribierbaren Moleküls. Außerdem hat sie den Nachteil, daß nur Templatnukleinsäuren mit einem definierten 3'-Ende nachgewiesen werden können.

Aufgabe der vorliegenden Erfindung war es daher, ein einfaches auf einer Transkriptionsreaktion beruhendes Amplifikationsverfahren zur Verfügung zu stellen, welches die Nachteiles des Standes der Technik vermeidet. Insbesondere sollte es in wenigen Schritten eine Produkt-RNA liefern und die bei der Transkription unter Verwendung von Promotorprimern auftretenden Hintergrundsignale verringern.

Diese Aufgabe wird durch die nachfolgend beschriebene Erfindung gelöst.

Gegenstand der Erfindung ist ein Verfahren zur spezifischen Herstellung einer Vielzahl von Ribonukleinsäuren unter Verwendung einer Templatnukleinsäure durch
- direkte Hybridisierung eines templatspezifischen Promotoroligonukleotids P1 und eines weiteren templatspezifischen Oligonukleotids P2 mit der Templatnukleinsäure T zu einem Komplex K und
- promotorgesteuerte Herstellung von Transkripten R, die die templatspezifischen Sequenzinformationen aus P1 und P2 enthalten,
wobei die eingesetzten Oligonukleotide P1 und P2 in keiner Verfahrensstufe miteinander enzymatisch ligiert werden. Ein weiterer Gegenstand ist ein Verfahren zum spezifischen Nachweis von Nukleinsäuren, welches auf dem erfindungsgemäßen Herstellungsverfahren beruht, und Reagenzien, die zur Durchführung der Verfahren geeignet sind.

Bei dem erfindungsgemäßen Verfahren handelt es sich beim Start der Reaktion und teilweise beim Nachweis der Reaktionsprodukte um eine spezielle Ausführungsform der sogenannten Hybridisierungstests, die in ihren Grundzügen dem Fachmann auf dem Gebiet der Nukleinsäurediagnostik bekannt sind. Soweit experimentelle Details im folgenden nicht ausgeführt sind, wird dazu vollinhaltlich auf "Nucleic acid hybridisation", Herausgeber B.D. Hames und S.J. Higgins, IRL Press, 1986, insbesondere in den Kapiteln 1 (Hybridisation Strategy), 3 (Quantitative Analysis of Solution Hybridisation) und 4 (Quantitative Filter Hybridisation), Current Protocols in Molecular Biology, Edt. F.M. Ausubel et al., J. Wiley and Son, 1987, insbesondere 2.9.1. - 2.9.10 und Molecular Cloning, Edt. J. Sambrook et al., CSH, 1989, insbesondere 9.4.7. - 9.5.8. Bezug genommen. Dazu gehören insbesondere die bekannten Methoden zur Herstellung von markierten Nukleosidtriphosphaten, wie sie auch in EP-A-0 324 474 beschrieben sind, die chemische Synthese von modifizierten und unmodifizierten Oligonukleotiden, die Spaltung von Nukleinsäuren mittels Restriktionsenzymen, die Auswahl von Hybridisierungs-bedingungen, durch welche eine Spezifität erreicht werden kann, die vom Ausmaß der Homologie zwischen den zu hybridisierenden Nukleinsäuren, deren GC-Gehalt und deren Länge abhängt, sowie die Bildung von Nukleinsäuren aus Deoxynukleosidtriphosphaten oder Ribonukleotidtriphosphaten mit Hilfe von Polymerasen, unter Verwendung von sogenannten Primern bzw. Promotor-Sequenzen.

Eine Markierung im Sinne der vorliegenden Erfindung besteht aus einer direkt oder indirekt nachweisbaren Gruppe L. Direkt nachweisbare Gruppen sind beispielsweise radioaktive (³²P), farbige, oder fluoreszierende Gruppen oder Metallatome. Indirekt nachweisbare Gruppen sind beispielsweise immunologisch oder enzymatisch wirksame Verbindungen, wie Antikörper, Antigene, Haptene oder Enzyme oder enzymatisch aktive Teilenzyme. Diese werden in einer nachfolgenden Reaktion oder Reaktionssequenz detektiert. Besonders bevorzugt sind Haptene, da mit ihnen markierte Nukleosidtriphosphate im allgemeinen besonders gut als Substrate von Polymerasen einsetzbar sind und eine anschließende Reaktion mit einem markierten Antikörper gegen das Hapten oder das haptenisierte Nukleosid leicht vorgenommen werden kann. Solche Nukleosidtriphosphate sind beispielsweise Brom-Nukleosidtriphosphate oder Digoxigenin, Digoxin- oder Fluorescein-gekoppelte Nukleosidtriphosphate. Als besonders geeignet haben sich die in EP-A-0 324 474 genannten Steroide und deren Detektion erwiesen. Für deren Inkorporation in Nukleinsäuren wird hiermit auf die EP-A-0 324 474 verwiesen.

Unter einem spezifischen Herstellungsverfahren oder Nachweis wird ein Verfahren verstanden, durch welches gewünschtenfalls selektiv bestimmte Nukleinsäuren auch in Gegenwart anderer Nukleinsäuren hergestellt bzw. nachgewiesen werden können. Es ist jedoch auch möglich, mehrere Nukleinsäuren oder eine Gruppe von Nukleinsäuren mit teilweise übereinstimmender oder ähnlicher Nukleotidsequenz oder mehrere Abschnitte einer Nukleinsäure in Gegenwart anderer Nukleinsäuren zum Gegenstand des Herstellungsverfahrens zu machen bzw. nachzuweisen. Zum Nachweis doppelsträngiger Nukleinsäuren kann jeder der beiden komplementären Stränge einbezogen werden. Unter einer im wesentlichen komplementären Nukleinsäure oder Nukleinsäuresequenz werden Nukleinsäuren oder Sequenzen verstanden, die mit der entsprechenden Nukleinsäure hybridisieren können, deren Nukleotidsequenz im hybridisierenden Bereich entweder genau komplementär zu der anderen Nukleinsäure ist oder sich in wenigen Basen von der genau komplementären Nukleinsäure unterscheidet. Die Spezifität richtet sich dabei sowohl nach dem Grad der Komplementarität als auch nach den Hybridisierungsbedingungen. Im wesentlichen zu einem Teil einer Templatnukleinsäure komplementäre Oligonukleotide werden im folgenden als templatspezifisch bezeichnet.

Grundlage der erfindungsgemäßen Verfahren sind Proben, welche Nukleinsäure gereinigt oder in Kombination mit anderen Bestandteilen, insbesondere anderen Nukleinsäuren, enthalten. Die Probe kann weitere Bestandteile wie Proteine, Salze, etc. enthalten. Mit dem erfindungsgemäßen Verfahren ist es möglich, Nukleinsäuresequenzen zu vervielfältigen, wenn diese Sequenzen in einer in der Probe enthaltenen Nukleinsäure vorhanden sind. Die Nukleinsäure, welcher die Herstellung von Ribonukleinsäuren zugrunde gelegt werden soll, wird im folgenden als Templatnukleinsäure (oder Template) bezeichnet.

Mit dem erfindungsgemäßen Verfahren können Nukleinsäure hergestellt werden, die die gesamte Nukleotidsequenzeninformation der Templatnukleinsäure, bevorzugt aber nur Teile davon enthalten. Zur Vermehrung von Teilsequenzen der Templatnukleinsäure ist es nicht erforderlich, jedoch möglich, die Templatnukleinsäure vor Durchführung des Verfahrens zu fragmentieren.

Die Templatnukleinsäure muß zur Durchführung des Verfahrens einzelsträngig vorliegen. Dies ist bei RNA normalerweise ohne weitere Vorbehandlung der Fall. Im Falle von DNA kann eine doppelsträngige Templatnukleinsäure durch Denaturierung auf einfache bekannte Weise einzelsträngig gemacht werden.

Ein Promotoroligonukleotid im Sinne der Erfindung ist eine Nukleinsäure, welche einen doppelsträngigen Bereich PRO enthält, welcher durch Erkennung und Bindung von RNA-Polymerase die Synthese von RNA startet. Dieser Bereich PRO enthält eine Sequenz, welche die Transkription des an diese Sequenz in downstream-Richtung anschließenden Nukleinsäurebereiches durch eine RNA-Polymerase initiiert. Die doppelsträngige Sequenz hat vorzugsweise eine Länge von 17-100 Basen, besonders bevorzugt 17-50 Basen. Geeignete doppelsträngige Sequenzen, die eine RNA Polymerase binden kann, sind beispielsweise in Nucleic Acids Research 12, Seite 7035-7056 (1984) und in Proteinsequences and DNA Analysis 1, Seite 269-280 (1988), Biophysical Chemistry, Part III, S. 1183-1211, Freeman & Co., San Francisco, 1980; J. Bacteriol 170, S. 5248-5256 (1988); Biochem. J. 224, S. 799-815 (1984); Gene Acal. Tech. 6, S. 29-32 (1989) EP-A-0 292 802 und Nucleic acid probes, ed Symons (CRC Press, Boca Raton, 1989) beschrieben. Die beiden Stränge des doppelsträngigen Anteils können entweder offen vorliegen oder upstream in Form einer Haarnadelstruktur verbunden sein. Der die komplementären Einzelstränge verbindende Haarnadelbereich (auch loop genannt) ist vorzugsweise 5-50 Nukleotide, besonders bevorzugt 5-10 Nukleotide lang und ist bevorzugt aus nur einer Nukleotidart aufgebaut. Am downstream 5'-Ende weist das Promotoroligonukleotid P1 einen einzelsträngigen templatspezifischen Bereich TEM1' auf. Der Bereich TEM1' ist vorzugsweise 6 bis 50 nt, besonders bevorzugt 12 bis 30 nt lang.

Der doppelsträngige Promotorbereich PRO kann von der templatspezifischen Sequenz TEM1' durch eine Sequenz von weiteren Nukleotiden getrennt sein.

Das 3'-Ende des Promotoroligonukleotids P1 ist bevorzugt nichtphosphoryliert, während das 5'-Ende phosphoryliert oder nichtphosphoryliert sein kann. Das downstream-3'-Ende von P1 kann auch gegen Verlängerung blockiert sein; z.B. durch ein Dideoxynukleotid. Das Promotoroligonukleotid P1 kann außerdem im downstream-Bereich anschließend an die Sequenz PRO eine weitere einzel- oder doppelsträngige Nukleotidsequenz enthalten. Bevorzugt sind transkriptionsfördernde Sequenzen (Nucl. Acids Res. 15, S. 8783-8798 (1987)). Die Region des Primers 1, die die zusätzlichen, die Transkription fördernden, wie bei J.F. Milligan et al. (1987), Nucl. Acids Res., 15, S. 8783 - 8798 beschriebenen, selbst- komplementären aber nicht Template-komplementären transkribierfähigen Sequenzen enthält, kann 1 - 20 Nukleotide lang sein; bevorzugt ist sie 5 Nukleotide (CCGCG) lang. Es können aber auch zusätzlich Nukleotidsequenzen zwischen PRO und TEM1'vorhanden sein, die weitere Reaktionsschritte (Startsequenzen für Replikation (ori-Sequenzen), Restriktionsschnittstellen, replizierbare Sequenzen, Bindungsstellen für Sequenzierprimer, Proteinbindungsstelle) ermöglichen.

Die Region zwischen Transkriptionsstart und TEM1'kann 0-150 nt lang sein. Das templatspezifische Oligonukleotid P2 enthält eine Nukleotidsequenz TEM 2', welche im wesentlichen komplementär zu einer weiteren Nukleotidsequenz der Templatnukleinsäure und daher mit dieser hybridisierbar ist. Diese Sequenz ist bevorzugt 6 bis 50 nt, besonders bevorzugt 12 bis 30 nt lang. Durch ihre Länge und Komplementarität läßt sich die Spezifität des erfindungsgemäßen Herstellungsverfahrens steuern. Durch geeignete Wahl dieser Sequenz ist es beispielsweise möglich, spezifisch nur eine oder auch mehrere Templatnukleinsäuren (z.B. Nukleinsäuren verschiedener Bakteriengenera oder Bakterienspezies in Gemischen) der Probe zum Gegenstand des erfindungsgemäßen Verfahren zu machen und ihre Sequenz zu vermehren.

In einem ersten Schritt des erfindungsgemäßen Verfahrens zur spezifischen Herstellung von Nukleinsäuren wird die Probe, welche die Templatnukleinsäure enthält, mit dem Promotorreagenz P1 und dem templatspezifischen Oligonukleotid P2 unter Hybridisierungsbedingungen umgesetzt. Dabei bildet sich ein transkribierbarer Nukleinsäurekomplex K aus, bei welchem die Templatnukleinsäure über einen doppelsträngigen Bereich TEM 1/TEM 1' an das Oligonukleotid P1 und über einen doppelsträngigen Bereich TEM 2/TEM 2' an das Oligonukleotid P2 hybridisiert ist. In downstream-Richtung können über weitere Bereiche TEM 3...TEM N weitere Oligonukleotide P3...PN an die Templatnukleinsäure anhybridisiert sein. Der Bereich TEM 1 ist von dem Bereich TEM 2 der Nukleinsäure verschieden. Bevorzugt liegen die Bereiche TEM 1' und TEM 2' direkt nebeneinander auf der Templatnukleinsäure, so daß sie nur durch ein nick getrennt sind. Im hybridisierten Zustand sind das 5'-Ende des Oligonukleotids P1 und das 3'-Ende des Primers P2 einander bevorzugt direkt benachbart oder 1-10, bevorzugt 1-5 Nukleotide voneinander entfernt, nicht jedoch miteinander kovalent verbunden. Sie können jedoch auch 1-150, bevorzugt 1-1000 nt voneinander entfernt sein. Bevorzugt wird dieser Gap dann zunächst durch eine Gap-Filling-Reaktion (DNA-Polymerase oder reverse Transkriptase, dNTPs) aufgefüllt, sodaß ein nick zurückbleibt. Der Primer P2 bzw. der im downstream-Bereich bindende Primer PN kann an seinem 5'-Bereich über die Sequenz TEM 2' bzw. TEM N' hinaus noch weitere Nukleotide enthalten, die nicht mit der Templatnukleinsäure hybridisieren. Eine solche Sequenz könnte z. B. eine ORI-Sequenz (Startregion für eine Replikation), eine replizierbare Sequenz, eine RE-Stelle, eine Sequenz zur Bindung eines Nukleinsäure-bindenden Proteins, eine homopolymere Sequenz oder eine weitere Promotorsequenz sein. P2...PN hat am 5'- und 3'-Ende bevorzugt eine OH-Gruppe. Die Hybridisierungsbereiche TEM 1 und TEM 2 sind bevorzugt jeweils 6-50 nt, besonders bevorzugt 12-30 nt lang.

Ein wesentlicher Gegenstand und Vorteil der Erfindung ist, daß anschließend an die Hybridisierungsreaktion von T, P1 und P2 im Gegensatz zu den bekannten Verfahren keine enzymatische Verknüpfung (Ligation) der Oligonukleotide P1 und P2 vorgenommen wird. Das erspart die Einstellung von Reaktionsbedingungen für die Ligasereaktion sowie insbesondere die Verwendung eines Ligationsenzyms. Voraussetzung ist jedoch, daß in der anschließenden Transkriptionsreaktion eine Transkriptase eingesetzt wird, die ungeachtet, ob der "Gegenstrang" (aus P1, P2, P3...PN) zu der Templatnukleinsäure einen oder mehrere nicks oder gaps aufweist, ein Transkript über die ganze Länge des Doppelstrangs vom Transkriptionsstartpunkt des Promotors bis zum 5'-Ende des Oligonukleotids P2 bzw. gegebenenfalls des Oligonukleotids PN bildet. Der Fachmann kann eine geeignete Transkriptase auf einfache Weise finden. Ein entsprechendes Experiment ist in Beispiel 3 geschildert. Eines der Enzyme, die für das erfindungsgemäße Verfahren geeignet sind, ist T7-RNA-Polymerase.

Der Nukleinsäurekomplex K wird nach seiner Bildung einer promotorgesteuerten enzymatischen Transkription unterworfen. Die Reaktionsbedingungen, unter denen eine promotorgesteuerte Transkription ablaufen kann, sind für diesen Komplex die gleichen wie für die Transkriptionsreaktionen des Standes der Technik. Sie hängen von dem gewählten Promotor/Polymerase-System ab. Beispiele für Promotorsysteme sind aus den Phagen T7, SP6 und T3 bekannt. Im wesentlichen sind eine RNA-Polymerase und Ribonukleosidtriphosphate (NTPs) erforderlich. Als besonders bevorzugtes Transkriptionssystem hat sich das Transkriptionssystem von T7 (T7-RNA-Polymerase und T7-Promotor) bewährt. Die verwendeten Polymerasen können auch thermostabil sein.

Die T7-RNA-Polymerase-promotorspezifischen komplementären Sequenzbereiche innerhalb der doppelsträngigen Region des Promotoroligonukleotids P1 können zwischen 12 und 20 Nukleotiden lang sein (kürzeste und längste funktionelle Promotorsequenz, die bei Milligan et al. (1987), Nucl. Acids Res., 15, S. 8783-8798 beschrieben ist). Bevorzugt hat der doppelsträngige Bereich eine Länge von 17 Nukleotiden.

Produkt der Transkription sind RNA-Transkripte R, deren 5'-Ende beim Transkriptionsstartpunkt und deren 3'-Ende durch die 5'-Endposition des Bereiches TEM 2 definiert wird. Bei Verwendung weiterer Oligonukleotide P3...PN enden die Transkripte bevorzugt mit einem der 5'-Endposition des entferntesten Oligonukleotids komplementären Ribonukleotid. Diese RNA enthält insbesondere die Sequenzen, die homolog zu TEM 1 und TEM 2 sind.

Im Falle einer alleinigen Hybridisierung von Primer 1 entsteht unabhängig von der Bindung an das Template ein kürzeres Transkript, welches bis zum 5'-Ende von Primer 1 reicht.

Das entstehende Transkript R kann das Endprodukt des erfindungsgemäßen Verfahrens zur Herstellung von Ribonukleinsäuren sein. Bevorzugt wird dieses Transkript jedoch in einem zyklisch verlaufenden Reaktionsprozeß weiter amplifiziert.

Da die Transkripte R homolog zu T sind und die Sequenzinformationen TEM 1 und TEM 2 enthalten, können sie bevorzugt wiederum Templatnukleinsäure zur Bildung eines transkribierbaren Komplexes K' aus R, P1 und P2 sein, welcher dann erneut transkribiert werden kann. So ist eine Amplifikation durch immer wieder durchgeführten Einsatz der Transkripte in die Reaktionsfolge
- Bildung von K'
- Transkription
möglich. Ein enormer Vorteil dieser Verfahrensführung ist, daß in keiner Phase eine Ligase eingesetzt werden muß.

Gegenüber Verfahren, in denen die Transkripte zunächst in cDNA umgeschrieben, diese mit einem Promotorprimer in einen transkribierbaren Komplex umgewandelt werden müssen und dieser transkribiert wird, besteht der Vorteil, daß die Bildung von cDNA entfallen kann; das erspart den Einsatz eines weiteren Enzyms.

Prinzipiell ist also erfindungsgemäß eine Bildung von Ribonukleinsäuren aus einer einzelsträngigen Templatnukleinsäure mittels nur eines einzigen Enzyms (einer promotorgesteuerten Polymerase) ohne Denaturierungsschritte zwischen den enzymatischen Reaktionsschritten zu erreichen. Temperaturzyklen oder zyklische Änderungen der Reaktionsbedingungen sind nicht erforderlich. Die Anzahl der Reaktionsschritte ist sehr gering.

Der genannte Zyklus kann so lange fortgeführt werden, bis die gewünschte Anzahl von Nukleinsäuren gebildet ist.

Die entstandenen Ribonukleinsäuren können auf bekannte Weise gereinigt oder/und weiterverarbeitet werden. Beispielsweise kann aus den Transkripten R cDNA hergestellt werden, z.B. unter Verwendung des Primers P2...PN.

Der Nachweis, ob eine ausreichende Zahl von Nukleinsäuren gebildet wurde, kann beispielsweise durch Zugabe einer nachweisbar markierten Detektorsonde, welche mit dem gewünschten Produkt hybridisieren kann, und Nachweis des Hybrids, oder durch direkten Nachweis der durch Einbau von nachweisbar markierten Mononukleotiden gebildeten Nukleinsäuren geschehen.

Ein Vorteil des erfindungsgemäßen Verfahrens ist, daß es isotherm geführt, d.h. bei einer Temperatur durchgeführt werden kann.

Ferner handelt es sich um eine templatspezifische Amplifikation und nicht nur um eine Signalverstärkung. Nur ein Satz Mononukleotide ist erforderlich (Ribonukleotide). Dies bedeutet geringere Herstellungskosten und keine gegenseitige Inhibierung der Polymerasen. Die Oligonukleotide sind leicht herstellbar und brauchen keine Blockierung ihrer 3'-Enden, da keine DNA-Polymerase vorhanden ist. Bei dem erfindungsgemäßen Verfahren sind keine definierten Enden für die Templatnukleinsäuren erforderlich; daher entfallen entsprechende Vorreaktionsschritte. Der Nachteil der LCR oder Repair Chain Reaction, daß die Gegenstrangoligonukleotide mit den spezifischen Oligonukleotiden kreuzhybridisieren, entfällt, da keine Gegenstrangoligonukleotide erforderlich sind. Die Reaktion ist dennoch im wesentlichen exponentiell, da die Transkripte jeweils wieder als Template wirken können.

In einer Ausführungsform des erfindungsgemäßen Verfahrens werden zur Stabilisierung der Einzelstrangkonfiguration im Bereich der Sequenzen TEM 1 und TEM 2 der Templatnukleinsäure blockierende Oligonukleotide BLO1 und BLO2 in den zu TEM 1 und TEM 2 benachbarten Bereichen BLO1' und BLO2' hybridisiert. Der Bereich BLO1' ist bevorzugt 2-10 Nukleotide upstream von TEM 1 entfernt lokalisiert. Der Bereich BLO2' sollte mehr als 10 Nukleotide downstream von der Bindungsstelle TEM 2 entfernt sein. Bevorzugt enthalten die Oligonukleotide BLO1 und BLO2 eine Modifikation zur Blockierung von Polymeraseaktivität am 3'-Ende, um Elongation zu verhindern, beispielsweise Dideoxyribonukleotide. Die Verwendung weiterer blockierender Oligonukleotide ist möglich.

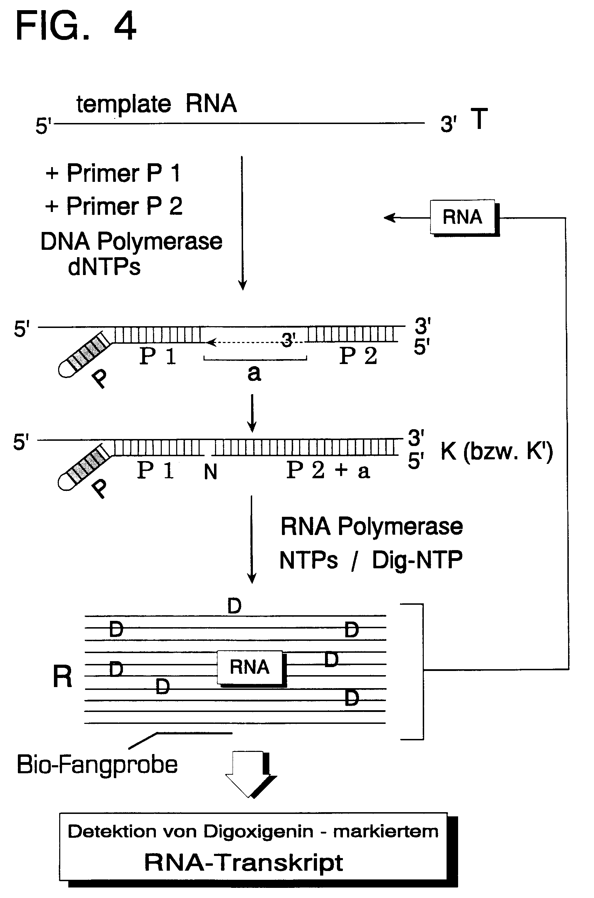

In einer weiteren Ausführungsform liegt TEM 2 1-150, bevorzugt 1-1000 nt vom 5'-Ende des Bereiches TEM 1 entfernt (a in Figur 4). Diese Lücke zwischen P1 und P2 kann durch eine RNA-abhängige oder DNA-abhängige DNA-Polymerase und dNTP im Ansatz aufgefüllt werden. Bevorzugt werden in diesem Fall 3'-blockierte Oligonukleotide P1 verwendet.

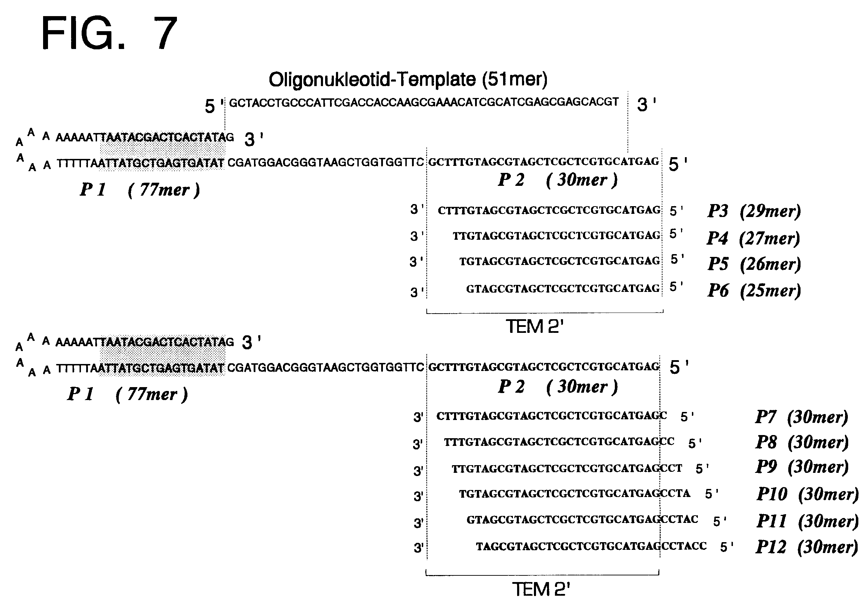

Beträgt diese Lücke bzw. dieses "gap" 1-10 Nukleotide, besonders bevorzugt 1-5 Nukleotide (Primer P3-P6 und Primer P7-P11 in Fig. 7) so kann diese von der T7 RNA Polymerase ohne vorherige Auffüllreaktion überlesen werden. Die Länge des dabei produzierten Transkripts entspricht der Summe aus den Längen der transkriptionsfähigen Sequenz des Primers 1 und der Länge des verwendeten Primers 2.

In einer weiteren, bevorzugten Ausführungsform wird daher die Sequenz des Primers 2 so gewählt, daß sich nach Anhybridisierung an das Template ein "gap" (s.o.) von 1-10, besonders bevorzugt 1-5 Nukleotiden zwischen dem 5'-Ende von Primer 1 und dem 3'-Ende von Primer 2 befindet. In Abwesenheit von DNA Polymeraseaktivität kann dieses "gap" während der Transkription durch die RNA Polymerase überlesen werden, d.h. im produzierten Transkript befinden sich das dem 5'-Ende von Primer 1 komplementäre Nukleotid und das dem 3'-Ende von Primer 2 komplementäre Nukleotid direkt benachbart. Das Transkript enthält in seiner Sequenz dann nur die Nukleotide, die dem transkribierbaren Bereich des Templates entsprechen, der in K doppelsträngig vorliegt.

Durch Anhybridisieren mehrerer verschiedener Primer an ein Template in Transkriptionsrichtung vom Promotoroligonukleotid 1 wird eine universelle Gensynthese bei gleichzeitiger Einführung von Mutationen durch "gaps" zwischen den einzelnen Primern möglich.

Ebenfalls Gegenstand der Erfindung ist daher ein Verfahren zur Herstellung von deletionsmutanten Nukleinsäuren mit Hilfe einer Templatnukleinsäure, enthaltend folgende Schritte:
- Hybridisierung mindestens eines Promotorprimers und eines Oligonukleotids, welche jeweils einen templatspezifischen Bereich aufweisen, wobei diese templatspezifischen Bereiche im wesentlichen zu Bereichen der Templatnukleinsäure komplementär sind, die 1-10 Nukleotide voneinander auf der Templatnukleinsäure gelegen sind und
- promotorgesteuerte Transkription.

Dieses Verfahren benutzt die Überleseeigenschaft von RNA-Polymerasen, insbesondere von T7-RNA-Polymerase. Unter einer deletionsmutanten Nukleinsäure wird in diesem Zusammenhang eine Nukleinsäure verstanden, welche im wesentlichen komplementär zu einer bestimmten Templat-Nukleinsäure ist, die sich jedoch von einem vollen Transkript dadurch unterscheidet, daß ein oder wenige Nukleotide fehlen. Der fehlende Bereich ist nicht ein solcher, der beim vollen Transkript direkt am 3'- oder 5'-Ende liegt.

Die zunächst erfindungsgemäß entstehenden deletionsmutanten Ribonukleinsäuren können auf bekannte Weise weiteren Reaktionen unterzogen werden (cDNA-Bildung oder erneute Umsetzung mit P1 und P2, die dann ohne gap mit der Nukleinsäure hybridisieren können).

Ein weiterer Gegenstand der Erfindung ist ein Verfahren zum spezifischen Nachweis von Nukleinsäure, welches das erfindungsgemäße Verfahren zur spezifischen Herstellung von Nukleinsäuren und seine Ausführungsformen einschließt, wobei die Transkripte R oder deren Folgeprodukte nachgewiesen werden. Unter Folgeprodukte ist hierbei insbesondere eventuell anschließend gebildete cDNA zu verstehen. Der Nachweis dieser Produkte kann prinzipiell auf bekannte Art und Weise geschehen, beispielsweise durch Hybridisierung mit markierten Sondennukleinsäuren und Nachweis markierter Hybride. Eine weitere einfache Methode ist der Einbau markierter Mononukleotide während der Transkriptionsreaktion oder/und die Auftrennung der Reaktionsprodukte in der Gelelektrophorese.

Eine besonders bevorzugte Ausführungsform des erfindungsgemäßen Verfahrens benutzt den Einbau eines detektierbar markierten Monoribonukleotids während der Transkriptionsreaktionen und die Hybridisierung mit einer Fangsonde, die entweder direkt an eine feste Phase gebunden ist oder bevorzugt durch Kopplung an eine chemische Gruppe wie z. B. Biotin immobilisierbar gemacht ist. Im Falle von immobilisierbaren Sonden ist eine anschließende Immobilisierung an eine Festphase, welche eine Bindeaffinität für die chemische Gruppe aufweist, möglich.

Bevorzugt wird nach Abtrennung der detektierbar markierten Mononukleotide die Markierung an der Festphase nachgewiesen. Als detektierbare Gruppe wird bevorzugt Digoxigenin-UTP (EP-A-0 324 744) oder Fluoreszein-UTP verwendet. Die Anwesenheit dieser Gruppe an der Festphase wird dann über einen enzymmarkierten Antikörper gegen Digoxigenin nachgewiesen.

Als Fang- oder Nachweissonde werden bevorzugt Sequenzen gewählt, welche homolog zu Teilsequenzen oder der Gesamtsequenz von P2 (oder ggf. PN) oder homolog zum 5'-Bereich von P1 und 3'-Bereich von P2 (jeweils ca. 10 nt) sind. Sie sind bevorzugt 6-5000 nt, besonders bevorzugt 12-50 nt lang. Bei der Auffüllreaktion liegen sie besonders bevorzugt im Bereich zwischen P1 und P2 (Auffüllbereich); daraus ergibt sich ein weiterer Vorteil für die Spezifität der Detektion.

In einer weitere Ausführungsform ist P2 mit einer zur Immobilisierung befähigten Gruppe markiert, bevorzugt am 5'-Ende. Über diese Gruppe können die Komplexe K und K' an eine Festphase zur Isolierung aus Gemischen gebunden werden.

Ebenfalls kann immobilisierbares P2 mit dem Transkript R hybridisiert und damit beispielsweise durch Detektion während der Transkription eingebauter nachweisbar markierter Mononukleotide detektiert werden. Dieses Vorgehen hat den Vorteil, daß keine weitere Fangsonde benötigt wird, da P2 als solche dienen kann.

In einer weiteren Ausführungsform ist P1 immobilisierbar modifiziert. Dann kann beispielsweise der Transkriptionskomplex K an eine Festphase gebunden und somit aus einem Gemisch von Nukleinsäuren getrennt werden.

Das erfindungsgemäße Nachweisverfahren hat alle Vorteile des erfindungsgemäßen Herstellungsverfahrens von Nukleinsäuren.

In den Figuren sind jeweils Nachweisverfahren beispielhaft gezeigt. Es handelt sich hier jedoch auch um erfindungsgemäße Herstellverfahren, wenn die Nachweisschritte weggelassen werden.

Ebenfalls Gegenstand der Erfindung sind Reagenzien und Reagenzkits zur Durchführung der erfindungsgemäßen Verfahren.

Gegenstand der Erfindung ist daher ein Reagenz, welches folgende Bestandteile enthält:
- ein templatspezifisches Promotoroligonukleotid P1, welches außer der doppelsträngigen Promotorsequenz PRO eine templatspezifische Sequenz TEM 1'enthält, und
- mindestens ein templatspezifisches Oligonukleotid P2.

Bevorzugt enthält das Reagenz außerdem mindestens eines der oben beschriebenen Oligonukleotide BLO1 und BLO2 oder weitere. Ebenfalls bevorzugt enthält es die vier Monoribonukleosidtriphosphatarten, unmodifiziert oder nachweisbar oder immobilisierbar modifiziert. Ferner kann es pH-Puffer und Hilfsstoffe, z.B. Stabilisatoren, enthalten, insbesondere solche, die für die Transkriptionsreaktion geeignet sind. Es enthält jedoch keine Ligase.

Weiterer Gegenstand ist ein Reagenzkit, welches in getrennten Behältern enthält:
1) das Promotoroligonukleotid P1, und das templatspezifische Oligonukleotid P2 und Monoribonukleosidtriphosphate; und
2) ein Transkriptionsenzym, passend zum Promotor.
3) keine Ligase
Das Reagenzkit kann die unter 1) genannten Bestandteile auch in getrennter Form enthalten.

Das Reagenzkit kann ferner Kontrollnukleinsäuren und/oder Reagenzien zur Probenherstellung enthalten.

Wenn das Reagenzkit zum Nachweis von Nukleinsäuren oder Nukleotidsequenzen verwendet werden soll, enthält es bevorzugt die dazu erforderlichen Reagenzien in einem getrennten Behälter, z.B. Fang- oder/und Nachweissonden.

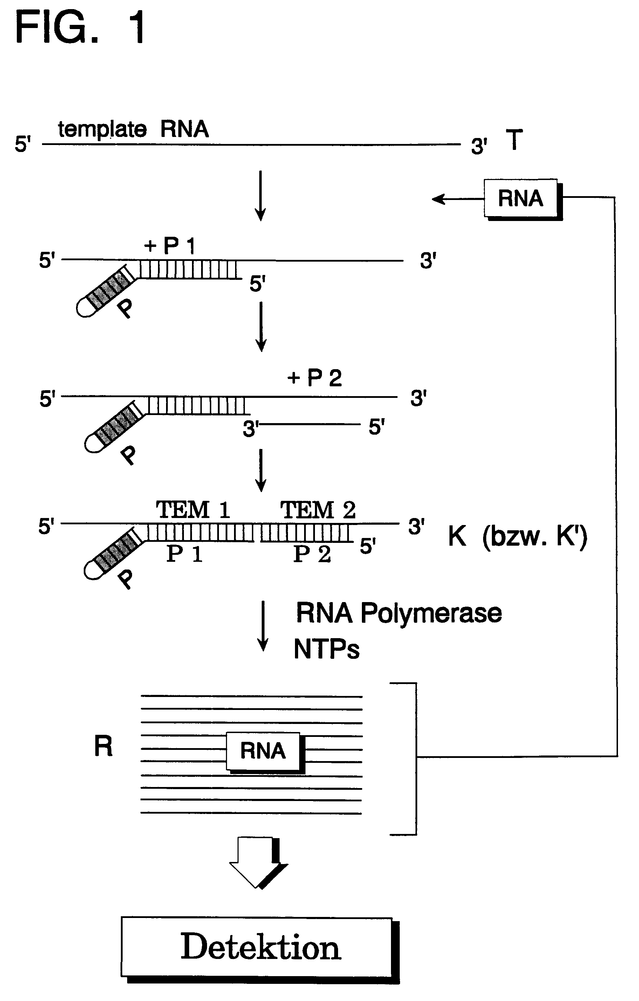

In Figur 1 ist das erfindungsgemäße Nachweisverfahren schematisch wiedergegeben. Die Oligonukleotide P1 und P2 können getrennt oder gemeinsam zugegeben werden. Bevorzugt ist ihre Menge und Konzentration gleich. Falls zu Beginn genügend P1 und P2 zugegeben wurde, ist eine Nachdosierung von P1 und P2 nicht nötig. Dasselbe gilt für die übrigen Reagenzien, insbesondere das Enzym und die Mononukleotide.

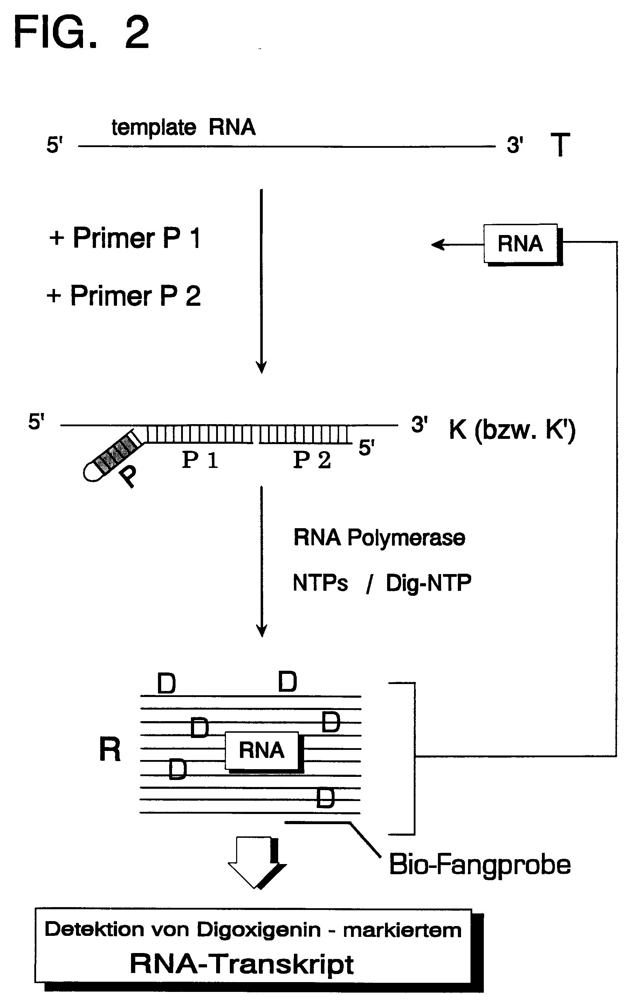

Figur 2 zeigt eine Ausführungsform des erfindungsgemäßen Nachweisverfahrens unter Einbau von nachweisbar markierten Monoribonukleosidtriphosphaten und anschließendes Abfangen der Transkripte mittels einer biotinylierten Fangsonde.

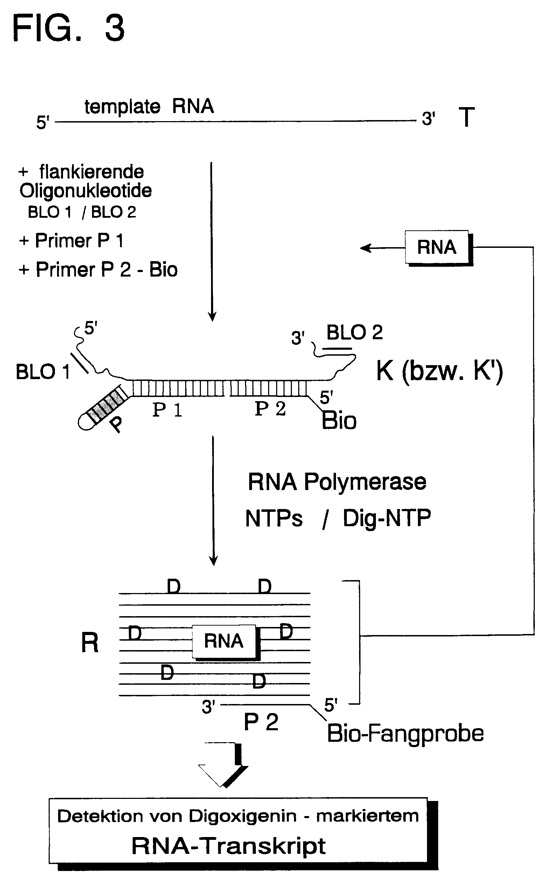

Figur 3 zeigt eine Ausführungsform, bei der sowohl Schutzoligonukleotide BLO1 und BLO2, als auch ein immobilisierbar substituiertes Oligonukleotid P2 verwendet werden. In dieser Ausführungsform wird ein Hybrid aus P2 und R nachgewiesen, welches sowohl nachweisbar als auch immobilisierbar markiert ist.

In Figur 4 wird eine Ausführungsform beschrieben, bei der nach Bildung des Nukleinsäurekomplexes K zunächst mittels DNA-Polymerase ein einzelsträngiger Bereich zwischen Oligonukleotid P1 und Oligonukleotid P2 aufgefüllt wird, bevor die Transkription durchgeführt wird. Die Fangsonde hybridisiert bevorzugt im aufgefüllten Bereich a.

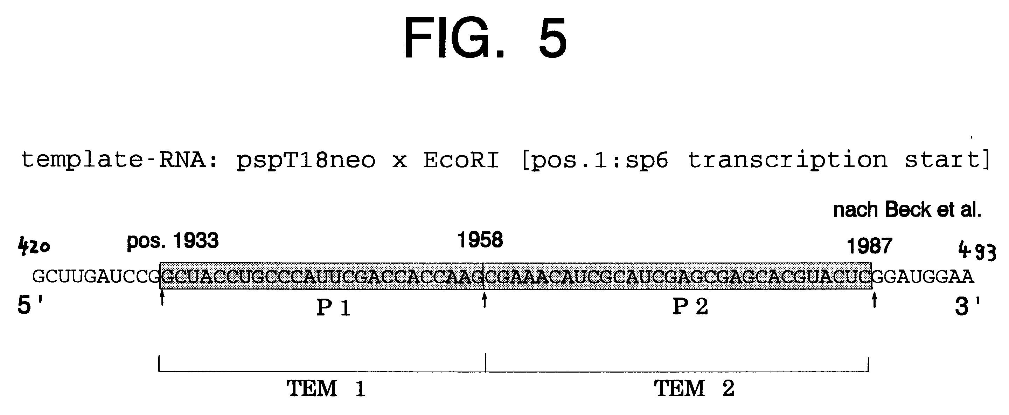

In Figur 5 ist der relevante Teil einer Templatnukleinsäure wiedergegeben. Es handelt sich um das Plasmid pSPT18neoxEco R1. Die Regionen, in welchen Oligonukleotid P1 und Oligonukleotid P2 anhybridisieren können, sind angegeben.

Figur 6 zeigt, wie ein chemisch synthetisiertes Templat, P1 und P2 von der Nukleotidsequenz her angeordnet sein können.

Figur 7 zeigt, wie ein Satz Oligonukleotide P3-P6 (bzw. P7-P12) zur Herstellung von gegenüber dem Template um bestimmte Nukleotide deletierten Transkripten dienen kann (durch unterschiedliche Länge der gap-Region zwischen Primer P1 und Primer P2).

### Bezugszeichenliste

- T: Templatnukleinsäure
- K: transkribierbarer Nukleinsäurekomplex aus P1, P2 und T
- K': transkribierbarer Nukleinsäurekomplex aus P1, P2 und R
- R: Transkript (RNA)
- P1: templatspezifisches Promotoroligonukleotid
- P2: templatspezifisches Oligonukleotid
- P: Promotorbereich (doppelsträngig) (entspricht PRO)
- TEM 1: proximal zum Transkriptionsstartpunkt auf der Templatnukleinsäure gelegene Sequenz
- TEM 2: distal zum Transkriptionsstartpunkt auf der Templatnukleinsäure gelegene Sequenz
- TEM 1': templatspezifischer Bereich von P1
- TEM 2': templatspezifischer Bereich von P2
- BIO: Biotin
- BLO1': Nukleotidbereich upstream von TEM 1
- BLO2': Nukleotidbereich downstream von TEM 2
- BLO1: Oligonukleotid komplementär zu BLO1'
- BLO2: Oligonukleotid komplementär zu BLO2'
- D: Digoxigenin
- a: Auffüllbereich
- NTP: Ribonukleosidtriphosphat
- dNTP: Desoxyribonukleosidtriphosphat
- N: Nick in Transkriptionseinheit

Die folgenden Beispiele erläutern die Erfindung näher.

### Beispiel 1

### Herstellung von RNA-Templates

Das Plasmid pSPT18 neo (Sequenz vgl. WO 89/06698) wird zur Herstellung von Transkripten des Neomycin-Resistenzgens (neo) verwendet. In pSPT18 wird das Neomycin-Gen (eine Aminoglycosid-3'-phosphotransferase II) wie in Beck et al. (1982), Gene, 19, 327 - 336, beschrieben, inseriert. Über das resultierende Plasmid pSPT18neo können mit SP6 RNA-Polymerase Transkripte des Gens in der Sense-Orientierung produziert werden. Plasmid pSPT18neo wird mit der Restriktionsendonuklease EcoRI linearisiert. Aus diesem linearisierten Plasmid werden durch in vitro Transkription, wie in Biochemicals for Molecular Biology, Boehringer Mannheim (1990), Seite 158, beschrieben, 1028 Nukleotide lange RNA-Transkripte hergestellt. Position 1 des Transkripts bezeichnet den SP6 RNA Polymerase Transkriptionsstartpunkt bzw. das erste Nukleotid des Transkripts. Nach Phenol-Extraktion zur Abreinigung enzymatischer Komponenten und Ethanol-Fällung können die so gereinigten Transkripte in den folgenden Beispielen als Templates eingesetzt werden.

### Beispiel 2

### Transkription an P1 (Promotorkonstrukt mit loop-Struktur)

### a) Herstellung von P1

P1 besteht aus einem Nukleinsäurestrang von 77 nt, wobei 25 nt an dessen 5'- Ende komplementär zu den Ribonukleotidpositionen 430 - 454 des in Beispiel 1 beschriebenen RNA-Transkripts bzw. zu den Nukleotidpositionen 1933 - 1957 der unter Beck et al. (s.o.) beschriebenen Sequenz, welche das neo-Gen enthält.

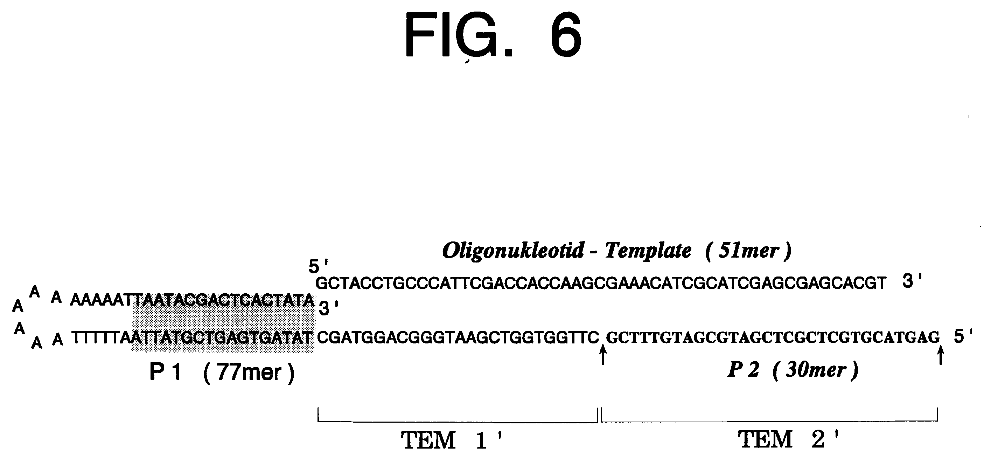

Zusätzlich enthält P1 die minimal notwendige selbstkomplementäre Sequenz des Promotors für die RNA-Polymerase des Bakteriophagen T7 (Sequenz vgl. P1 in Fig. 6) (J.F. Milligan et al. (1987), Nucl. Acids Res., Vol. 15, No. 21, 8783-8798). Diese selbst-komplementären Sequenzen sind durch eine AT-reiche Region voneinander getrennt, die die Ausbildung des partiellen Doppelstrangs in Lösung fördert. Außerdem kann P1 zusätzliche, die Transkription fördernde, wie bei J.F. Milligan et al. (1987), Nucl. Acids Res., 15, S. 8783 - 8798 beschrieben, selbst-komplementäre aber nicht Template- komplementäre transkribierfähige Sequenzen enthalten.

Das P1 entsprechende DNA Oligonukleotid von 77 Nukleotiden wird nach der Synthese in einem automatisierten DNA Synthesizer über ein 20%iges denaturierendes Polyacrylamid-Gel durch Elektrophorese, wie in Molecular Cloning (1989), Editors Sambrook, Fritsch, Maniatis, CSH, Seiten 6.39 - 6.48 beschrieben, gereinigt. Um ein möglichst vollständiges Annealing der selbstkomplementären Sequenzen von P1 zu ermöglichen, wird dieses DNA Oligonukleotid nach der Reinigung in einem Reaktionsgefäß 10 Minuten auf 90°C erhitzt und anschließend 10 Minuten auf Eis abgekühlt. P1 wird auf seine Fähigkeit, in Gegenwart von T7 RNA Polymerase unter den nachfolgend beschriebenen Versuchsbedingungen RNA-Transkripte zu produzieren, untersucht.

### b) Transkriptionsreaktion

Das Reaktionsgemisch enthält in einem Endvolumen von 25 µl:
40 mmol/l Tris-HC1 (pH 8.0 bei 37°C), 6 mmol/l MgCl₂, 10 mmol/l NaCl, 10 mmol/l Dithiothreitol (DTT), 2 mmol/l Spermidin-HCl, 5 % (v/v) Polyethylenglycol MW 6000, 0,01 % (v/v)Triton-X-100, je 2 mmol/l ATP, UTP, GTP, CTP (pH 8.0 bei 37°C), 5 µCi [ 32P]-CTP (400 Ci/mmol, Amersham), 500 nmol/l Primer 1, 15 U/µl T7 RNA Polymerase (Boehringer Mannheim), 1 U/µl RNAse Inhibitor (Boehringer Mannheim)
Die nicht enzymatischen Einsatzstoffe werden vor der Verwendung mit 0,01% Diethylpyrocarbonat, wie in Molecular Cloning (s.o.) Seiten 7.3 - 7.4 beschrieben, behandelt.

In einem Reaktionsgefäß von 100 µl Inhalt werden die einzelnen Komponenten gemischt und der Ansatz eine Stunde bei 37°C inkubiert.

### c) Detektion

Anschließend wird die Reaktion durch Zugabe des gleichem Volumens Formamid- Stoppuffer (95% Formamid, 25 mM EDTA, 0,01% Xylencyanol, 0,01% Bromphenolblau), 3 minütiges Erhitzen auf 68°C und Abkühlung des Reaktionsansatzes auf Eis gestoppt. Ein Aliquot des denaturierten Reaktionsansatzes wird dann auf ein 7 M Harnstoff, 12% Polyacrylamid-Gel einer Schichtdicke von 0,8 mm aufgetragen. Die Gelelektrophorese wird analog U.K. Laemmli (1970), Nature, 277, S. 680-685 durchgeführt. Das Gel wird anschließend autoradiographiert und die radioaktiven Produkte analysiert.

Die Reaktion kann ebenfalls durch Zugabe von 10 mmol/l EDTA und 0,1% SDS gestoppt werden. Zur Detektion werden die Transkriptionsprodukte dann, wie in Molecular Cloning (s.o.), Seiten 7.43 - 7.45 beschrieben, in einem 1,5%igem denaturierendem Agarosegel aufgetrennt und die Reaktionsprodukte durch Färbung in einer Acridiniumorange-Lösung (5 µg/ml) sichtbar gemacht.

Wird dem Reaktionsansatz kein [ 32P]-CTP zugegeben, können die spezifischen Reaktionsprodukte, nach Gelelektrophorese in Polyacrylamid-Gelen über Northern-Blotting auf eine Nylonmembran transferiert und UV-fixiert, durch in-situ Hybridisierung mit komplementären, radioaktiv oder nichtradioaktiv markierten (Biochemicals for Molecular Biology, s.o., S. 112 - 115) DNA Oligonukleotiden detektiert werden. Diese Art von Hybridisierungen sind bei J. Meinkoth and G. Wahl (1984), Anal.Biochem., 138, S. 267-284, und in Nucleic Acid Hybridisation" (1985) Editors B.D. Hames and S.J. Higgins, IRL Press, Oxford, S. 139 -159 beschrieben.

Ebenfalls können die Reaktionsprodukte, nach Gelfiltration mit einer Sephadex G-50 Säule von nichteingebautem [ 32P]-CTP getrennt, durch Ethanol-Fällung konzentriert und durch Auftropfen auf eine Nylonmembran, UV-Fixierung und Exposition eines Röntgenfilms mit der getrockneten Membran über die resultierende Schwärzung des Films detektiert werden.

Durch den Einbau von nichtradioaktiv markierten NTPs statt [ 32P]-CTP können die Produkte im DOT-, SLOT- oder Northern-blot direkt sichtbar gemacht werden. Der Einbau von Digoxigenin-11-UTP bzw. Biotin-16-UTP (vgl. WO 89/06698) kann zum direkten Nachweis mit Anti-Digoxigenin-AP-Konjugat bzw. mit Streptavidin-AP-Konjugat verwendet werden.

Die Detektion wird durch Reaktion von Alkalischer Phosphatase mit dem entsprechenden Substrat 5-bromo-4-hloro-3-indoyl phosphat (X-Phosphat) und nitroblueetrazolium salt (NBT), durch Farbumschlag der Reaktionslösung wie in Biochemicals for Molecular Biology (s.o.) S. 109 - 115 beschrieben, oder über eine durch Alkalische Phosphatase vermittelte Chemilumineszenzreaktion mit 3-(2'Spiroadamantan)-4-methoxy-4-(3''- phosphoryloxy)-phenyl-1,2-dioxetan (AMPPD , Boehringer Mannheim), wie bei I. Bronstein and P. McGrath (1989), Nature, 338, S. 599 - 600, beschrieben, ermöglicht.

Die Sensitivität der Chemilumineszenzreraktion kann durch Zusatz von 5,6 umol/l 5-N- tetradecanoylaminofluorescein (Fluoreszenzenhancer) in 0,75 mol/l 2-amino-2-methyl-1-propanol-Puffer, pH 9,6, wie bei M. Musani et al. (1991) Anal.Biochem., 194, S. 394 - 398 beschrieben, noch erhöht werden. Die Dokumentation dieser Lichtemission durch Chemilumineszenz erfolgt über die Belichtung eines Polaroid-oder Röntgenfilms.

Es wird RNA in der Länge vom Transkriptionsstart am Promotor bis zum 5'-Ende von P1 produziert.

### Beispiel 3

### Transkription des Hybrids aus P1, P2 und RNA-Template bzw. Oligonukleotid- Template

### a) Herstellung von P2 und des Oligonukleotid-Templates

Die Sequenz von P2 (DNA Oligonukleotid von 30 Nukleotiden; Sequenz vgl. Fig. 6) ist komplementär zu den Ribonukleotidpositionen 456 - 485 des in Beispiel 1 beschriebenen RNA-Transkripts bzw. zu den Nukleotidpositionen 1958 - 1987 der unter Beck et al. (s.o.) beschriebenen Sequenz, welche das neo-Gen enthält. Diese komplementäre Sequenz ist so gewählt, daß das 3'-Ende des Primers 2 unmittelbar benachbart zum 5'-Ende von P1 mit dem RNA-Template hybridisieren kann. Das P2 entsprechende DNA Oligionukleotid von 30 Nukleotiden wird nach der Synthese, wie in Beispiel 2 beschrieben, gereinigt.

Die Sequenz des Oligonukleotid-Templates (DNA Oligonukleotid von 51 Nukleotiden; Sequenz vgl. Fig. 6) ist homolog zu den Ribonukleotidpositionen 430 - 481 des in Beispiel 1 beschriebenen RNA-Transkripts. Diese zum RNA-Template homologe Sequenz ist so gewählt, daß die Templatekomplementären Bereiche von P1 und P2 direkt benachbart an das Oligonukleotid-Template hybridisieren.

### b) Transkriptionsreaktion

Um ein möglichst vollständiges Annealing der komplementären Sequenzen von P1 und P2 an die Template-Nukleinsäure zu ermöglichen, werden äquimolare Mengen dieser DNA Oligonukleotide zusammen mit der Template-Nukleinsäure vor der Zugabe der übrigen Reaktionskomponenten in einem Reaktionsgefäß 10 Minuten auf 90°C erhitzt und 10 Minuten auf Eis abgekühlt. Anschließend werden die denaturierten DNA Oligonukleotide zur Ausbildung der hairpin-Struktur von P1
10 Minuten bei 37°C inkubiert. Der Transkriptionspuffer und die enzymatischen Komponenten (s. Beispiel 2) werden in einem Reaktionsgefäß gemischt und die vorinkubierten Oligonukleotide P1 und P2 in einer Endkonzentration von je 500 nmol/l zugegeben. Der Reaktionsansatz wird eine Stunde bei 37°C inkubiert.

Die Transkriptionsreaktion wird wie in Beispiel 2 durchgeführt.

Anschließend werden die Reaktionsprodukte, wie in Beispiel 2 beschrieben, detektiert und die RNA-Produkte analysiert.

Es wird RNA in der Länge vom Transkriptiosstart am Promotor innerhalb von P1 bis zum 5'-Ende von P2 produziert.

### Beispiel 4

Transkription der Hybride aus Primer P1, einem der Primer P2 bis P6 und RNA-Template bzw. Oligonukleotide-Template.
a) Herstellung der Primer 3-6 (einzelsträngig, linear)
   Die Sequenz des Primers P3 (DNA Oligonukleotid von 29 Nukleotlden; Sequenz vgl. Fig. 7) ist komplementär zu den Ribonukleotidpositionen 457-485 des in Beispiel 1 beschriebenen RNA-Transkripts bzw. zu den Nukleotidpositionen 1959-1987 der unter Beck et al. (s.o.) beschriebenen Sequenz.
   Die Sequenz des Primers P4 (DNA Oligonukleotid von 27 Nukleotiden; Sequenz vgl. Fig. 7) ist komplementär zu den Ribonukleotidpositionen 459-485 des in Beispiel 1 beschriebenen RNA-Transkripts bzw. zu den Nukleotidpositionen 1961-1987 der unter Beck et al. (s.o.) beschriebenen Sequenz.
   Die Sequenz des Primers P5 (DNA Oligonukleotid von 26 Nukleotiden; Sequenz vgl. Fig. 7) ist komplementär zu den Ribonukleotidpositionen 460-485 des in Beispiel 1 beschriebenen RNA-Transkripts bzw. zu den Nukleotidpositionen 1962-1987 der unter Beck et al. (s.o.) beschriebenen Sequenz.
   Die Sequenz des Primers P6 (DNA Oligonukleotid von 25 Nukleotiden; Sequenz vgl. Fig. 7) ist komplementär zu den Ribonukleotidpositionen 461-485 des in Beispiel 1 beschriebenen RNA-Transkripts bzw. zu den Nukleotidpositionen 1963-1987 der unter Beck et al. (s.o.) beschriebenen Sequenz.
   Diese komplementären Sequenzen sind so gewählt, daß die 3'-Enden der Primer P3-P6 nicht unmittelbar benachbart zum 5'-Ende des Primers P1 mit dem RNA-Template hybridisieren können. Die den Primern P3-P6 entsprechenden DNA Oligonukleotide werden nach der Synthese, wie in Beispiel 2 beschrieben, gereinigt.
   Die Hybridisierungsprodukte aus Primer P1, Primer P3-P6 und RNA-Template bzw. Oligonukleotid-Template werden auf die Fähigkeit der T7 RNA Polymerase hin getestet, die entstandenen "gaps" zwischen Primer P1 und Primer P3-P6 im kodierenden Nukleinsäurestrang bei der Transkription zu überlesen.
b) Transkriptionsreaktion
   Die Transkriptionsreaktin wird wie in Beispiel 2 durchgeführt.
   Um ein möglichst vollständiges Annealing der komplementären Sequenzen der Primer P1 und P3-P6 an die Template-Nukleinsäure zu ermöglichen, werden äquimolare Mengen dieser DNA Oligonukleotide vor der Zugabe der übrigen Reaktionskomponenten in einem Reaktionsgefäß 10 Minuten auf 90°C erhitzt und 10 Minuten auf Eis abgekühlt. Anschließend werden die denaturierten DNA Oligonukleotide 10 Minuten bei 37°C hybridisiert.
   Der Transkriptionspuffer und die enzymatischen Komponenten (s. Beispiel 2) werden in einem Reaktionsgefäß gemischt und die Oligonukleotide P1 und P3-P6 ineiner Endkonzentration von je 500 nmol/l zugegeben. Der Reaktionsansatz wird eine Stunde bei 37°C inkubiert.
   Anschließend werden die Reaktionsprodukte, wie in Beispiel 2 beschrieben, detektiert und die RNA-Produkte analysiert.
   Es wird RNA in der Länge vom Transkriptionsstart am Promotor innerhalb des Primers P1 bis zum 5'-Ende der Primer P3-P6 produziert. Die Länge der produzierten Transkripte beträgt 54 nt (bei Primer P3), 52 nt (bei Primer P4), 51 nt (bei Primer P5) und 50 nt bei Primer P6 im Ansatz.
   Die fehlenden Nukleotide im Bereich des "gap" werden überlesen und RNA Transkripte in der Länge des doppelsträngigen Bereichs im transkriptionsfähigen Komplex K produziert.

### Beispiel 5

Transkription der Hybride aus Primer P1, P7 bis P12 und RNA-Template bzw. Oligonukleotid-Template.
a) Herstellung der Primer P7-P12 (einzelsträngig, linear)
   Die Sequenz des Primers P7 (DNA Oligonukleotid von 30 Nukleotiden; Sequenz vgl. Fig. 7) ist komplementär zu den Ribonukleotidpositionen 457-486 des in Beispiel 1 beschriebenen RNA-Transkripts bzw. zu den Nukleotidpositionen 1959-1988 der unter Beck et al. (s.o.) beschriebenen Sequenz.
   Die Sequenz des Primer P8 (DNA Oligonukleotid von 30 Nukleotiden; Sequenz vgl. Fig. 7) ist komplementär zu den Ribonukleotidpositionen 458-487 des in Beispiel 1 beschriebenen RNA-Transkripts bzw. zu den Nukleotidpositionen 1960-1989 der unter Beck et al. (s.o.) beschriebenen Sequenz.
   Die Sequenz des Primers P9 (DNA Oligonukleotid von 30 Nukleotiden; Sequenz vgl. Fig. 7) ist komplementär zu den Ribonukleotidpositionen 459-488 des in Beispiel 1 beschriebenen RNA-Transkripts bzw. zu den Nukleotidpositionen 1961-1990 der unter Beck et al. (s.o.) beschriebenen Sequenz.
   Die Sequenz des Primers P10 (DNA Oligonukleotid von 30 Nukletodien; Sequenz vgl. Fig. 7) ist komplementär zu den Ribonukleotidpositionen 460-489 des in Beispiel 1 beschriebenen RNA-Transkripts bzw. zu den Nukleotidpositionen 1962-1991 der unter Beck et al. (s.o.) beschriebenen Sequenz.
   Die Sequenz des Primers P11 (DNA Oligonukleotid von 30 Nukleotiden; Sequenz vgl. Fig. 7) ist komplementär zu den Ribonukleotidpositionen 461-490 des in Beispiel 1 beschriebenen RNA-Transkripts bzw. zu den Nukleotidpositionen 1963-1992 der unter Beck et al. (s.o.) beschriebenen Sequenz.
   Die Sequenz des Primers P12 (DNA Oligonukleotid von 30 Nukleotiden; Sequenz vgl. Fig. 7) ist komplementär zu den Ribonukleotidpositionen 462-491 des in Beispiel 1 beschriebenen RNA-Transkripts bzw. zu den Nukleotidpositionen 1964-1993 der unter Beck et al. (s.o.) beschriebenen Sequenz.
   Diese komplementären Sequenzen sind so gewählt, daß die 3'-Enden der Primer P7-P12 nicht unmittelbar benachbart zum 5'-Ende des Primers P1 mit dem RNA-Template hybridisieren können. Die den Primer P7-P12 entsprechenden DNA Oligonukleotide werden nach der Synthese, wie in Beispiel 2 beschrieben, gereinigt.
   Die Hybridisierungsprodukte aus Primer 1, Primer 7-12 und RNA-Template bzw. Oligonukleotid-Template werden auf die Fähigkeit der T7 RNA Polymerase hin getestet, die entstandenen "gaps" zwischen Primer 1 und Primer 7-12 im codierenden Nukleinsäurestrang bei der Transkription zu überlesen und RNA Transkripte in der Länge des doppelsträngigen Bereichs im transkriptionsfähigen Komplex K zu produzieren.
b) Transkriptionsreaktion
   Die Transkriptionsreaktion wird wie in Beispiel 2 durchgeführt.
   Um ein möglichst vollständiges Annealing der komplementären Sequenzen der Primer 1 und Primer 7-12 an die Template-Nukleinsäure zu ermöglichen, werden äquimolare Mengen dieser DNA Oligonukleotide zusammen mit der Template-Nukleinsäure vor der Zugabe der übrigen Reaktionskomponenten in einem Reaktionsgefäß 10 Minuten auf 90°C erhitzt und 10 Minuten auf Eis abgekühlt. Anschließend werden die DNA Oligonukleotide 10 Minuten bei 37°C inkubiert.
   Der Transkriptionspuffer und die enzymatischen Komponenten (s. Beispiel 2) werden in einem Reaktionsgefäß gemischt und die Oligonukleotide Primer 1 und Primer 7-12 in einer Endkonzentration von je 500 mmol/l zugegeben. Der Reaktionsansatz wird eine Stunde bei 37°C inkubiert.
   Anschließend werden die Reaktionsprodukte, wie in Beispiel 2 beschrieben, detektiert und die RNA-Produkte analysiert.
   Es wird RNA in der Länge vom Transkriptionsstart am Promotor innerhalb des Primer 1 bis zum 5'-Ende der Primer 7-12 produziert. Die Länge der produzierten Transkripte beträgt bei allen Ansätzen (mit den Primern 7-12) 55 nt.
   Die fehlenden Nukleotide im Bereich des einzelnen "gap" werden überlesen und die Transkription stoppt oder intiiert auch nicht in diesem Bereich.

### Beispiel 6

### Bandbreite der Reaktion

Im Folgenden werden Reaktionsbedingungen angegeben, innerhalb derer das erfindungsgemäße Verfahren mit Erfolg durchgeführt werden kann. Der Fachmann kann damit aufgrund weniger Versuche jedoch auch hiervon abweichende Bedingungen ermitteln.

| | optimale Bedingungen | Bandbreite |
|---|---|---|
| Tris-HCl | 40 mM (pH 8.0) | 2 - 150 mM (pH 7.5 -8.5) |
| MgC12 | 6 mM | 2 - 20 mM |
| NaCl | 10 mM | 0 - 200 mM |
| DTT | 10 mM | 2 - 20 mM |
| Spermidin-HCl | 2 mM | 0 - 10 mM |
| PEG 6000 | 5 % | 2 - 10 % |
| Triton-X-100 | 0.01 % | 0.01 - 0.5 % |
| BSA | - | 0 - 100 g/ml |
| RNAse Inhibitor | 1 U//ul | 0 - 5 U//ul |
| NTPs | 2 mM | 0.2 - 5 mM |
| Primer 1/2/3 | 500 nM | 100 nM - 1.5 /uM |
| Primer 4/5 | - | 0 - 1,5 /umol |
| RNA - Polymerasen | T7 RNA Polymerase | T7, SP6, T3 RNA, N4 |
| | | Polymerasen |
| T7 RNA Polymerase | 15 U/µl | 5 - 25 U/µl |
| Reaktionstemperatur | 37°C | 35 - 42°C |
| Reaktionszeit | 60 min. | 20 min. - 3 Std. |
| Reaktionsvolumen | 25 /ul | 20 - 200 /ul |
| Vorhybridisierung von | 0-30 min. | 0-6 h |

## Patentansprüche

1. Verfahren zur spezifischen Herstellung einer Vielzahl von Ribonukleinsäuren unter Verwendung einer Templatnukleinsäure T durch
- direkte Hybridisierung eines templatspezifischen Promotoroligonukleotids P1 und eines weiteren templatspezifischen Oligonukleotids P2 mit der Templatnukleinsäure T zu einem Komplex K und
- promotorgesteuerte Herstellung von Transkripten R, die zumindest die templatspezifischen Sequenzinformationen aus P1 und P2 enthalten,
dadurch gekennzeichnet, daß die eingesetzten Oligonukleotide P1 und P2 in keiner Verfahrensstufe miteinander enzymatisch ligiert werden.

2. Verfahren zur Herstellung von deletionsmutanten Nukleinsäuren mit Hilfe einer Templatnukleinsäure, enthaltend folgende Schritte:
- Hybridisierung mindestens eines Promotorprimers und eines Oligonukleotids, welche jeweils einen templatspezifischen Bereich aufweisen, wobei diese templatspezifischen Bereiche im wesentlichen zu Bereichen der Templatnukleinsäure komplementär sind, die 1-10 Nukleotide voneinander auf der Templatnukleinsäure gelegen sind und
- promotorgesteuerte Transkription.

3. Verfahren zum spezifischen Nachweis einer Templatnukleinsäure durch
- direkte Hybridisierung eines templatspezifischen Promotoroligonukleotids P1 und eines weiteren templatspezifischen Oligonukleotids P2 mit der Templatnukleinsäure T zu einem Komplex K und
- promotorgesteuerte Herstellung von Transkripten R, die zumindest die templatspezifischen Sequenzinformationen aus P1 und P2 enthalten, sowie
- Nachweis der Transkripte R,
dadurch gekennzeichnet, daß die eingesetzten Oligonukleotide P1 und P2 in keiner Verfahrensstufe miteinander enzymatisch ligiert werden.

4. Verfahren nach einem der Ansprüche 1 bis 3, dadurch gekennzeichnet, daß
- die Transkripte R mit P1 und P2 zu einem Komplex K' hybridisiert werden und
- K' ebenfalls promotorgesteuert transkribiert wird.

5. Verfahren nach einem der Ansprüche 1 bis 3, dadurch gekennzeichnet, daß bei der Transkription nachweisbar oder immobilisierbar markierte Monoribonukleosidtriphosphate in die Transkripte R eingebaut werden.

6. Verfahren nach Anspruch 5, dadurch gekennzeichnet, daß P2 immobilisiert oder immobilisierbar ist.

7. Reagenz zur Herstellung von Ribonukleinsäuren aus Templatnukleinsäuren enthaltend:
- ein templatspezifisches Promotoroligonukleotid P1, welches außer der doppelsträngigen Promotorsequenz PRO eine templatspezifische Sequenz TEM 1'enthält, und
- mindestens ein templatspezifisches Oligonukleotid P2.
- Keine Ligase.

8. Reagenzkit zur Herstellung von Ribonukleinsäuren enthalten in getrennten Behältern
- das Promotoroligonukleotid P1, und das templatspezifische Oligonukleotid P2 und Monoribonukleosidtriphosphate; und
- ein Transkriptionsenzym, passend zum Promotor
welches keine Ligase enthält.


SEQUENZPROTOKOLL

(1) ALLGEMEINE INFORMATION:

(i) ANMELDER:
(A) NAME: Boehringer Mannheim GmbH
(B) STRASSE: Sandhoferstr. 116
(C) ORT: Mannheim
(E) LAND: Deutschland
(F) POSTLEITZAHL: 6800
(G) TELEPHON: 06217594348

(ii) ANMELDETITEL: Verfahren zur spezifischen Herstellung von
Ribonukleinsaeuren

(iii) ANZAHL DER SEQUENZEN: 14

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(v) GEGENWÄRTIGE ANMELDEDATEN:
ANMELDENUMMER: EP 92116113.9

(2) INFORMATION ZU SEQ ID NO: 1:

(i) SEQUENZ CHARAKTERISTIKA:
(A) LÄNGE: 51 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 1:

GCTACCTGCC CATTCGACCA CCAAGCGAAA CATCGCATCG AGCGAGCACG T 51

(2) INFORMATION ZU SEQ ID NO: 2:

(i) SEQUENZ CHARAKTERISTIKA:
(A) LÄNGE: 77 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 2:

CTTGGTGGTC GAATGGGCAG GTAGCTATAG TGAGTCGTAT TAATTTTTAA AAAAAAAAAT 60

TAATACGACT CACTATA 77

(2) INFORMATION ZU SEQ ID NO: 3:

(i) SEQUENZ CHARAKTERISTIKA:
(A) LÄNGE: 30 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 3:

GAGTACGTGC TCGCTCGATG CGATGTTTCG 30

(2) INFORMATION ZU SEQ ID NO: 4:

(i) SEQUENZ CHARAKTERISTIKA:
(A) LÄNGE: 73 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 4:

GCUUGAUCCG GCUACCUGCC CAUUCGACCA CCAAGCGAAA CAUCGCAUCG AGCGAGCACG 60

UACUCGGAUG GAA 73

(2) INFORMATION ZU SEQ ID NO: 5:

(i) SEQUENZ CHARAKTERISTIKA:
(A) LÄNGE: 29 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 5:

CTTTGTAGCG TAGCTCGCTC GTGCATGAG 29

(2) INFORMATION ZU SEQ ID NO: 6:

(i) SEQUENZ CHARAKTERISTIKA:
(A) LÄNGE: 27 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 6:

TTGTAGCGTA GCTCGCTCGT GCATGAG 27

(2) INFORMATION ZU SEQ ID NO: 7:

(i) SEQUENZ CHARAKTERISTIKA:
(A) LÄNGE: 26 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 7:

TGTAGCGTAG CTCGCTCGTG CATGAG 26

(2) INFORMATION ZU SEQ ID NO: 8:

(i) SEQUENZ CHARAKTERISTIKA:
(A) LÄNGE: 25 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 8:

GTAGCGTAGC TCGCTCGTGC ATGAG 25

(2) INFORMATION ZU SEQ ID NO: 9:

(i) SEQUENZ CHARAKTERISTIKA:
(A) LÄNGE: 30 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 9:

CTTTGTAGCG TAGCTCGCTC GTGCATGAGC 30

(2) INFORMATION ZU SEQ ID NO: 10:

(i) SEQUENZ CHARAKTERISTIKA:
(A) LÄNGE: 30 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 10:

TTTGTAGCGT AGCTCGCTCG TGCATGAGCC 30

(2) INFORMATION ZU SEQ ID NO: 11:

(i) SEQUENZ CHARAKTERISTIKA:
(A) LÄNGE: 30 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 11:

TTGTAGCGTA GCTCGCTCGT GCATGAGCCT 30

(2) INFORMATION ZU SEQ ID NO: 12:

(i) SEQUENZ CHARAKTERISTIKA:
(A) LÄNGE: 30 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 12:

TGTAGCGTAG CTCGCTCGTG CATGAGCCTA 30

(2) INFORMATION ZU SEQ ID NO: 13:

(i) SEQUENZ CHARAKTERISTIKA:
(A) LÄNGE: 30 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 13:

GTAGCGTAGC TCGCTCGTGC ATGAGCCTAC 30

(2) INFORMATION ZU SEQ ID NO: 14:

(i) SEQUENZ CHARAKTERISTIKA:
(A) LÄNGE: 30 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 14:

TAGCGTAGCT CGCTCGTGCA TGAGCCTACC 30